# EUROPEAN PATENT APPLICATION

(11) **EP 4 316 577 A1**
(43) Date of publication of application: **07.02.2024**
(21) Application number: 22774731.8
(22) Date of filing: 09.02.2022
(51) Int. Cl.: A61N 5/06

(54) **DEVICE FOR SUPPLYING LIGHT AND HEAT**

(30) Priority: 23.03.2021 JP 2021048581
(71) Applicant: Kibe Planning Co., Ltd., Tokyo 143-0016 (JP)
(72) Inventor: KIBE, Machiko, Tokyo 1430016 (JP); MORI, Kazumi, Kawasaki-shi, Kanagawa 2140032 (JP)
(74) Representative: Schicker, Silvia
(86) International application number: PCT/JP2022/005041
(87) International publication number: WO 2022/201930

(57) **Abstract**

The object of the present invention is to provide an apparatus for feeding light and heat that has a simple structure, is easy to use, and is capable of supplying light of near-infrared rays while appropriately warming the subcutaneous area. The apparatus (1) for feeding light and heat comprises a light source holder (10) for a human body that can be fixed in contact with the human body, and has a cavity (18) for the human body that is recessed from a side that contacts the human body, a light source (16) for the human body that is installed in the cavity for the human body, a light source holder (20) for temperature control, wherein a cavity (28) for temperature control is formed from one side, a light source (26) for temperature control that is installed in the cavity for the temperature control, a temperature sensor (50) installed at the entrance of the cavity for temperature control of the light source holder for temperature control, a power source (30) supplying predetermined power to the light source for the human body and the light source for temperature control, and a controller (40) that adjusts the power supplied to the light source for the human body and the light source for temperature control based on a temperature measured by the temperature sensor.

## Description

### Technical Field

The present invention relates to an apparatus for feeding light and heat for irradiating and warming a human body.

### Background Art

During cancer treatment, lymph node resection, lymph vessel rupture, radiotherapy, use of taxane drugs, etc., cause obstruction or failure of lymphatic channels, poor lymphatic flow, and accumulation of body fluids, resulting in long-term retention. This condition is called the development of lymphedema. Lymphedema is a retained state of protein-rich excess fluid, which further enhances water holding ability. Limbs with lymphedema tend to have lower body temperatures than healthy limbs. The reason is considered as follows. The subcutaneous high internal pressure also adversely affects blood circulation, leading to compressive ischemia. Sustained compressive ischemia leads to cell degeneration, atrophy, and fibrosis. These reductions in energy production, compressive ischemia, etc., become the basis for poor blood circulation, and are factors in lowering body temperature in limbs with lymphedema.

Compression therapy is currently widely used. For the purpose of reducing edema (excessive body fluid), stretch elastic bandages are wrapped around the limbs with lymphedema many times (multiple layers) and a strong external pressure of 40 mmHg or more is applied to move the excess body fluid or to promote reabsorption of water into capillaries. There are also shrinkable garments (sleeves, stockings) for the same compression purpose. In addition, skin care to protect the epithelial connective tissue (skin) of limbs with lymphedema, which is always exposed to high internal pressure, and maintenance of joint mobility and muscle stretching may be used to maintain blood circulation and lymphatic flow. The patient will do a combination of these on a daily basis. Even if excessive body fluid is reduced, protein fluid remains under the skin. That is, the current treatment is mainly "symptomatic treatment," and no treatment aiming at complete cure has been known. When lymphedema develops, it is difficult to cure completely, and it is necessary to carry out regular treatment mainly based on self-management for the rest of the patient's life, and thus, the physical, mental, and economic burden on the patient is heavy.

Increased internal pressure by edema causes internal pressure stress to nearby cells, which leads to a vicious cycle of modulation of the regularity of physiological mechanisms and destabilization of homeostatic functions in humans. In its defense, the internal pressure stress on the cells should be reduced, and it is necessary to promote an increase in the energy produced by the cells. For that purpose, activation of cytochrome C oxidase is effective. The cytochrome C oxidase is known to be a photoreceptor for light of 600 nm in the visible range to near-infrared rays, and has been reported to be activated by light irradiation (for example, see Non-Patent Literature 1). Furthermore, near-infrared rays penetrate to a depth of 2 to 6 mm under the skin, and near-infrared irradiation is effective in activating cytochrome C oxidase.

At the same time, it is also effective to enhance blood circulation by warming the cells to about 40°C to promote the supply of nutrients and oxygen on the arterial side and the reabsorption of body fluids on the venous side. That is, in order to aim at remission of lymphedema, it is preferable to apply near-infrared rays while warming the subcutaneous area.

On the other hand, a therapeutic device using light irradiation has also been known (see Patent Literature 1, for example). Patent Literature 1 discloses a device in which LEDs having different wavelengths are arranged in layers in the main body of the device that is made of neoprene (registered trademark). In this device, the main body of the device having a different shape is attached to each treatment site, such as a hand, arm, or leg, to irradiate the affected area with light. The device also has a sensor for measuring skin surface temperature, which is attached to the main body of the device together with the processor.

The device disclosed in Patent Literature 1 requires a different main body of the device for each site to be worn. In addition, a sensor and a processor are attached to the main body of the device, which makes the device complicated and large-scale. Furthermore, the sensor measures the skin surface temperature and does not measure the subcutaneous temperature that is the target of treatment, and thus the subcutaneous warming cannot necessarily be performed appropriately.

Accordingly, it is an object of the present invention to provide an apparatus for feeding light and heat that has a simple structure, is easy to use, and is capable of supplying light of near-infrared rays while appropriately warming the subcutaneous area.

### Prior-art Publication

### Patent Literature

[Patent Literature 1]
U.S. Patent Application Publication No. US2005/0177093

### Non-Patent Literature

[Non-Patent Literature 1]
Kushihiki, et al., "Bioeffects of low-power lasers," Journal of Japan Society for Laser Surgery and Medicine, 2014, Vol. 34, No. 4, pp. 384-393

### Disclosure of Invention

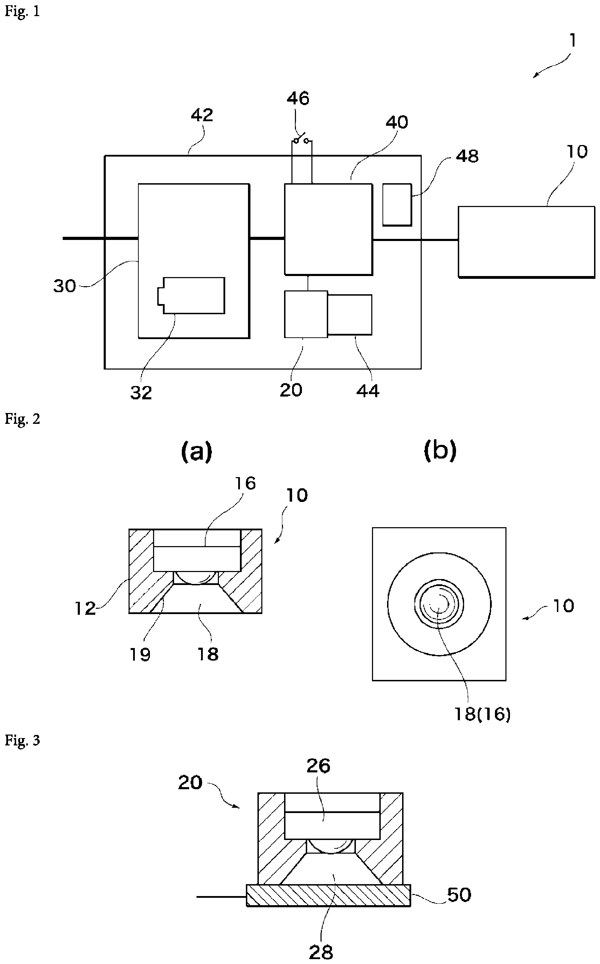

In order to solve the abovementioned problem, as shown in Figs. 1 - 3, for example, an apparatus 1 for feeding light and heat of a first aspect of the present invention comprises a light source holder 10 for a human body that can be fixed in contact with the human body, and has a cavity 18 for the human body that is recessed from a side that contacts the human body. It also comprises a light source 16 for the human body that is installed in the cavity 18 for the human body and emits light of near-infrared rays. It also comprises a light source holder 20 for temperature control, wherein a cavity 28 for temperature control is formed from one side, wherein the light source holder 20 for temperature control is formed of the same material as the light source holder 10 for the human body, and wherein the cavity 28 for temperature control has the same shape as the cavity 18 for the human body. It also comprises a light source 26 for temperature control installed in the cavity 28 for temperature control and emitting light of the same wavelength as the light source 16 for the human body. It also comprises
a temperature sensor 50 installed at the entrance of the cavity 28 for temperature control of the light source holder 20 for temperature control. It also comprises a power source 30 that supplies a same power to the light source 16 for the human body and the light source 26 for temperature control. It also comprises a temperature switch 46 that is operated when subcutaneous temperature reaches a suitable temperature. It also comprises a controller 40 that adjusts power supplied from the power source 30 to the light source 16 for the human body and the light source 26 for temperature control based on a temperature measured by the temperature sensor 50. In the apparatus 1 the controller 40 controls the power supplied to the light source 16 for the human body and the light source 26 for temperature control so that the temperature measured by the temperature sensor 50 is maintained at the temperature when the temperature switch 46 is operated.

With this configuration, the light source for the human body irradiates the human body with light to warm the subcutaneous area, and a temperature sensor measures the energy of the light emitted from the light source to provide the energy to the skin, and thus the energy to be supplied to the skin. Since the energy of the light to be supplied can be adjusted based on the subcutaneous temperature, the apparatus for feeding light and heat can be obtained, by which the structure of the apparatus is made simple and easy to use, and light of near-infrared rays can be supplied while appropriately warming the subcutaneous area.

In order to solve the abovementioned problem, as shown in Figs. 1-3, for example, an apparatus 1 for feeding light and heat of a second aspect of the invention comprises a light source holder 10 for a human body capable of being fixed in contact with the human body, wherein a cavity 18 for the human body that is recessed from a side in contact with the human body is formed. It also comprises a light source 16 for the human body that is installed in the cavity 18 for the human body and emits light of near-infrared rays. It also comprises a light source holder 20 for temperature control, wherein a cavity 28 for temperature control is formed from one side, wherein the light source holder 20 for temperature control is formed of the same material as the light source holder 10 for the human body, and wherein the cavity 28 for temperature control has the same shape as cavity 18 for the human body. It also comprises a light source 26 for temperature control installed in the cavity 28 for temperature control and emitting light of the same wavelength as the light source 16 for the human body. It also comprises a temperature sensor 50 that is located at an entrance of the cavity 28 for temperature control of the light source holder 20 for temperature control and that covers the entrance. It also comprises a power source 30 providing a same power to the light source 16 for the human body and the light source 26 for temperature control. It also comprises a memory 48 for storing a set temperature, which is a target temperature of a temperature measured by the temperature sensor 50. It also comprises a controller 40 that adjusts power supplied from the power source 30 to the light source 16 for the human body and the light source 26 for temperature control based on the temperature measured by the temperature sensor 50. In the apparatus 1 the controller 40 adjusts the power supplied to the light source 16 for the human body and the light source 26 for temperature control to maintain the temperature measured by the temperature sensor 50 at the set temperature.

With this configuration, the light source for the human body irradiates the human body with light to warm the subcutaneous area. The temperature sensor measures the energy of the light emitted from the light source so that the energy of the light to be supplied can be adjusted based on the energy to be supplied to the skin, eventually the subcutaneous temperature. Therefore, the apparatus for feeding light and heat can be obtained, by which the structure of the apparatus is made simple and easy to use, and light of near-infrared rays can be supplied while appropriately warming the subcutaneous area.

In an apparatus 1 for feeding light and heat of a third aspect of the present invention, the light of the same wavelength has a peak wavelength of 800 nm to 820 nm. With this configuration, since the human body is irradiated with light having a peak wavelength of 800 nm to 820 nm, the energy reaches a depth of 2 to 6 mm under the skin, i.e., the subcutaneous layer, and cytochrome C oxidase can be activated.

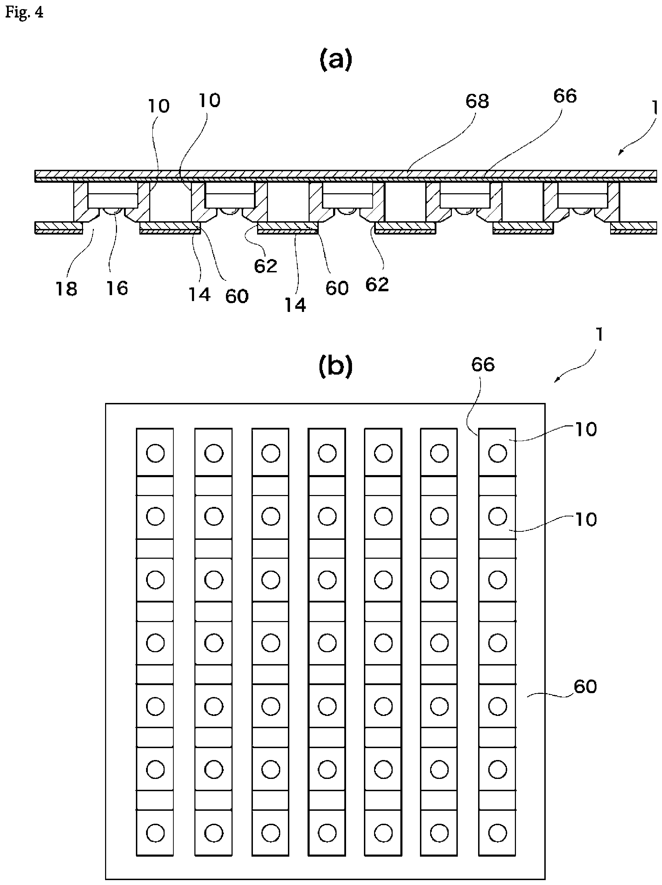

In an apparatus 1 for feeding light and heat of a fourth aspect of the present invention, as shown in Fig. 4, for example, the light source holder 10 for the human body is made of silicon rubber and is replaceable. With this configuration, since the light source holder for the human body is made of silicone rubber, even if the skin perspires, it does not absorb sweat and dries easily, disinfection is easy, and the specific gravity is relatively heavy, so that when worn it is adaptable to the complex shape of limbs with lymphedema. Furthermore, it is hygienic because the light source holder for the human body that contacts the human body is replaceable.

An apparatus 1 for feeding light and heat of a fifth aspect of the present invention, as shown in Fig. 4, for example, has a plurality of the light source holders 10 for the human body, wherein the plurality of light source holders 10 for the human body are fixed to a sheet 60 and arranged two-dimensionally. With this configuration, since the plurality of light source holders for the human body are fixed to the sheet and arranged two-dimensionally, light and heat can be supplied to a wide range. Also, since the light source holders for the human body are fixed to the sheet, it is easy to attach the apparatus for feeding light and heat along the shape of the limbs with lymphedema.

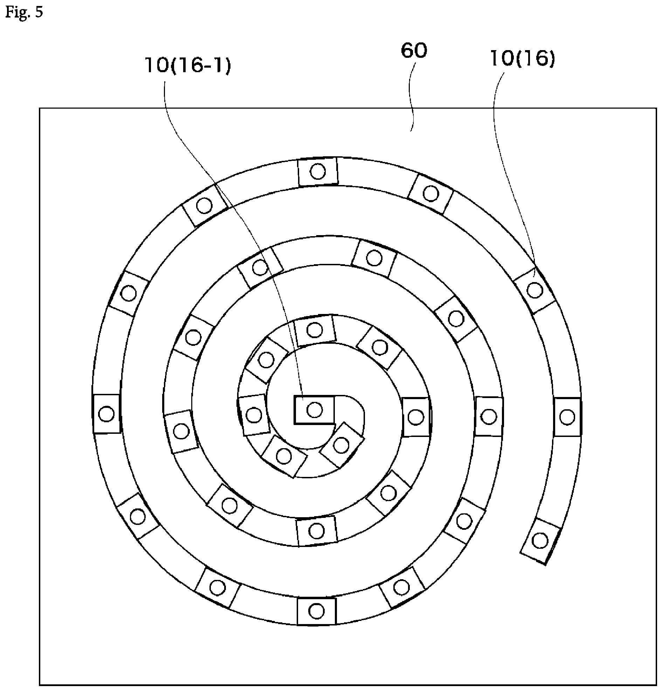

In an apparatus 1 for feeding light and heat of a sixth aspect of the present invention, as shown in Fig. 5, for example, the plurality of light source holders 10 for the human body are arranged in whorl. With this configuration, since the light source holders for the human body are arranged in whorl, it can supply light and heat that is strong at the position corresponding to the center and gradually weakens around it. Thus, light and heat can be supplied with a peak at the center.

In an apparatus 1 for feeding light and heat of a seventh aspect of the present invention, as shown in Fig. 4, for example, the plurality of light source holders 10 for the human body are arranged in a lattice pattern, and the light source holders 10 for the human body in a same row are connected by a connector 66 that is less bendable than the sheet. With this configuration, since the apparatus for feeding light and heat can be easily wound in a direction orthogonal to the longitudinal direction of the connector, for example, it can be easily wound around an arm or a leg. Furthermore, by running the cable supplying power to the light source holder for the human body along the connector, the cable is protected and does not get in the way during use.

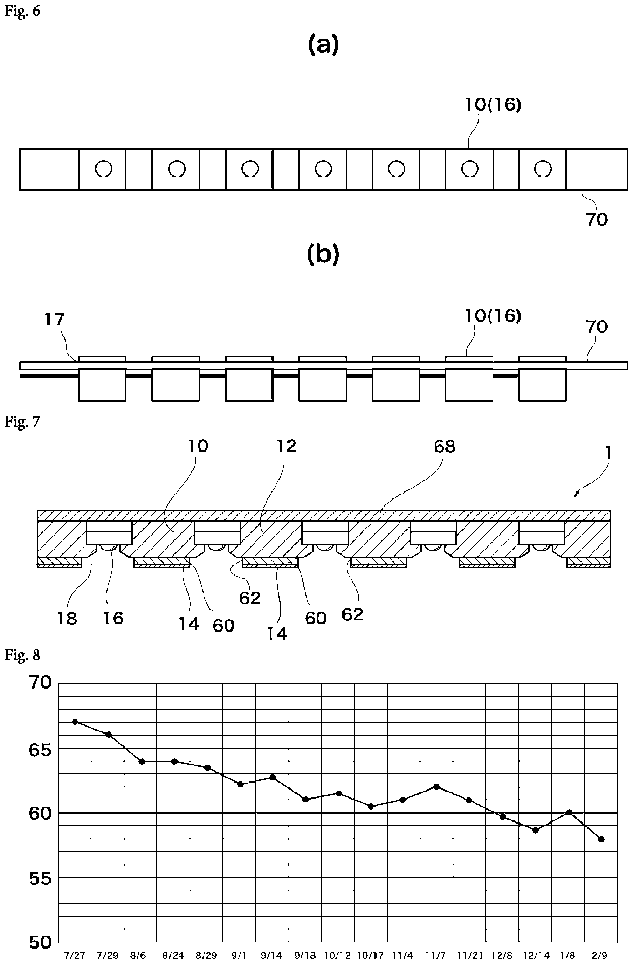

In an apparatus 1 for feeding light and heat of an eighth aspect of the present invention, as shown in Fig. 7, for example, the plurality of cavities 18 for the human body are formed in the light source holder 10 for the human body, and each cavity 18 for the human body is provided with the light source 16 for the human body. With this configuration, since the plurality of light source holders for the human body are fixed to the sheet and arranged two-dimensionally, light and heat can be appropriately supplied subcutaneously at desired positions, and the sheet can be bent to make it easy to wear along the complex shape of the human body.

An apparatus 1 for feeding light and heat of a ninth aspect of the present invention, as shown in Fig. 6, for example, has a plurality of the light source holders 10 for the human body, wherein the plurality of the light source holders 10 for the human body are movably attached to a belt 70. With this configuration, since the apparatus for feeding light and heat can be attached to the human body by winding the belt, and the plurality of the light source holders for the human body can be moved, light and heat can be adequately delivered to desired positions under the skin.

By the present invention, the light source for the human body irradiates the human body with light to warm the subcutaneous region. The temperature sensor measures the energy of the light emitted from the light source to be provided to the skin. Thus, based on the energy provided to the skin, eventually the subcutaneous temperature, the supplied light can be adjusted, so the apparatus for feeding light and heat that has a simple structure and can supply light of near-infrared rays while adequately warming the subcutaneous region can be provided.

The basic Japanese patent application, No. 2021-048581, filed March 23, 2021, is hereby incorporated by reference in its entirety in the present application.

The present invention will become more fully understood from the detailed description given below. However, that description and the specific embodiments are only illustrations of the desired embodiments of the present invention, and so are given only for an explanation. Various possible changes and modifications will be apparent to those of ordinary skill in the art on the basis of the detailed description.

The applicant has no intention to dedicate to the public any disclosed embodiment. Among the disclosed changes and modifications, those which may not literally fall within the scope of the present claims constitute, therefore, under the doctrine of equivalents, a part of the present invention.

The use of the articles "a," "an," and "the" and similar referents in the specification and claims are to be construed to cover both the singular and the plural form of a noun, unless otherwise indicated herein or clearly contradicted by the context. The use of any and all examples, or exemplary language (e.g., "such as") provided herein, is intended merely to better illuminate the invention, and so does not limit the scope of the invention, unless otherwise stated.

### Brief explanation of the drawings

[Fig. 1]
   Fig. 1 is a structural drawing explaining an apparatus for feeding light and heat as an embodiment of the present invention.
[Fig. 2]
   Fig. 2 is an explanatory drawing of a light source holder for a human body and a light source for the human body. Fig. 2 (a) is a cross sectional view and (b) is a plan view taken from the skin side.
[Fig. 3]
   Fig. 3 is a cross sectional view explaining of a light source holder for temperature control, a light source for temperature control, and a temperature sensor.
[Fig. 4]
   Fig. 4 shows an example of fixing the light source holder for a human body to a seat. Fig. 4(a) is a cross sectional view and (b) is a plan view showing an example for an arrangement of the light sources for the human body.
[Fig. 5]
   Fig. 5 is a plan view of another exemplary arrangement of the light sources for the human body.
[Fig. 6]
   Fig. 6 shows an example of attaching the light source holders for a human body to a belt. Fig. 6(a) is a plan view and (b) is a cross sectional view.
[Fig. 7]
   Fig. 7 is a cross sectional view of an example in which a plurality of cavities for the human body are formed on an expanded light source holder for a human body, each equipped with light sources for the human body, respectively.
[Fig. 8]
   Fig. 8 is a graph showing an exemplary result of medical treatment using an apparatus for feeding light and heat of the present invention.

### Description of Embodiments

Below, with reference to the drawings, embodiments of the present invention are discussed. In the drawings, the same or corresponding members are denoted by the same reference numbers. Thus, duplicate descriptions are omitted. First, with reference to Fig. 1, the overall structure of an apparatus 1 for feeding light and heat is discussed.

The apparatus 1 for feeding light and heat has a light source holder 10 for a human body that can be fixed in contact with the human body. As discussed below, the light source holder 10 for the human body can be fixed by wrapping around the human body such as an arm and a leg. When the light source holder 10 for the human body is made of silicone rubber, it does not absorb perspiration from the skin and dries easily. It is also preferable because it has an appropriate weight for mounting the light source holder 10 for the human body along the complicated shape of the human body. However, it is not limited to be made of silicon rubber, and may be formed of, for example, chloroprene rubber.

Now, with reference to Fig. 2, the light source holder 10 for the human body is discussed in detail. A cavity 18 for the human body is formed in the light source holder 10 for the human body to recess from the side that contacts the human body. An LED shelf 19 for mounting and fixing an LED 16, which is a light source for the human body, is formed inside the cavity 18 for the human body. The light source 16 for the human body is placed on the LED shelf 19 so that the LED 16 can irradiate the human body with light. The cavity 18 for the human body may be formed through the light source holder 10 for the human body. By forming the cavity 18 for the human body through the light source holder 10 for the human body, it is easy to mount the LED 16 on the LED shelf 19 from the back side of the light source holder 10 for the human body (the side opposite to contacting the human body) so that manufacturing is facilitated. Note that the light source 16 for the human body may not be an LED, but be an optical fiber or any other device that can irradiate the human body with light. The light source 16 for the human body emits light with a wavelength of near-infrared rays, preferably with a peak wavelength of 800 nm to 820 nm, as will be discussed below.

The light source 16 for the human body installed in the cavity 18 for the human body irradiates the human body P with light of near-infrared rays. It is typically an LED, but may be optical cable or other light source. The effect of applying light of near-infrared rays to the human body will be explained separately. The light source 16 for the human body is placed at a predetermined distance from the human body with which the apparatus 1 for feeding light and heat is in contact. The predetermined distance is preferably within the range of 3 mm to 5 mm, for example, but it is not particularly limited. If it is too close to the skin of the human body, it may come into contact with the skin and cause burns. If it is too far apart, there may be a disadvantage such that the transmission of light energy will be poor, or the light source holder 10 for the human body will become large.

Returning to FIG. 1, the apparatus 1 for feeding light and heat has the light source holder 20 for temperature control. If the light source holder 20 for temperature control is accommodated in a control box 42 for controlling the apparatus 1 for feeding light and heat, the overall structure is compact and convenient to use.

Now, with reference to Fig. 3, the light source holder 20 for temperature control is discussed in detail. The light source holder 20 for temperature control is preferably made of the same material as the light source holder 10 for the human body. A cavity 28 for temperature control is formed in the light source holder 20 for temperature control, and the cavity 28 for temperature control is provided with a light source 26 for temperature control. The cavity 28 for temperature control is preferably of the same shape and size as the cavity 18 for the human body. Also, the light source 26 for temperature control is preferably the same as the light source 16 for the human body. It should be noted that the "same" here is not strictly the same. For example, if it is the same model number, or manufactured based on the same specification or the same drawing, it is the same, and if there are differences due to aging etc., it is the same.

A temperature sensor 50 is installed at the entrance of the cavity 28 for temperature control opposite the light source 26 for temperature control. The temperature sensor 50 may be a thermistor or other known temperature sensor. The temperature sensor 50 preferably has a black outer surface to enhance absorption of light energy. Further, the temperature sensor 50 covers the entrance of the cavity 28 for temperature control to absorb all the light energy generated from the light source 26 for temperature control which attempts to dissipate from the cavity 28 for temperature control. That is, the temperature sensor 50 can measure the light energy delivered to the human body from the light source 16 for the human body.

Returning again to Fig. 1, the apparatus 1 for feeding light and heat has a power source 30 that supplies power to the light source 16 for the human body and the light source 26 for temperature control. The power source 30 may be a battery or a converter that converts from home electrical power. The same power is supplied to the light source 16 for the human body and the light source 26 for temperature control. Thus, the light source 16 for the human body and the light source 26 for temperature control emit light with the same intensity.

The apparatus 1 for feeding light and heat has a controller 40. The controller 40 adjusts the amount of power supplied to the light source 16 for the human body and the light source 26 for temperature control based on the temperature measured by the temperature sensor 50.

The apparatus 1 for feeding light and heat has a temperature switch 46. When the subcutaneous temperature of a user (not shown) wearing the apparatus 1 for feeding light and heat becomes a suitable temperature due to the irradiation from the light source 16 for the human body, the user or an operator (not shown) of the apparatus 1 for feeding light and heat actuates or operates the temperature switch 46. When the subcutaneous temperature becomes a suitable temperature while using the apparatus 1 for feeding light and heat, it means that the subcutaneous area is appropriately warmed and blood circulation is promoted. It is typically 40°C to 42°C, but there are individual differences. Especially in limbs with lymphedema, the body temperature tends to be lower than in healthy limbs. For example, even if it is warmed to a normal temperature of 37°C, blood circulation is promoted, and the subcutaneous temperature may become a suitable temperature. For example, blood flow in the capillaries may be measured to operate the temperature switch 46 when the blood flow increases. Alternatively, the user (not shown) wearing the apparatus 1 for feeding light and heat may operate the temperature switch 46 when the subcutaneous temperature is warmed, blood circulation is promoted, and he or she feels comfortable like bathing. Therefore, the controller 40 adjusts the amount of power supplied to the light source 16 for the human body and the light source 26 for temperature control so as to maintain the state in which the temperature switch 46 is operated, that is, so that the temperature measured by the temperature sensor 50 is the same as the temperature when the temperature switch 46 was operated. The amount of power can be adjusted by providing a thermostat 44 and turning on/off the power supply to the light source 16 for the human body and the light source 26 for temperature control. Alternatively, the voltage applied to the light source 16 for the human body and the light source 26 for temperature control may be adjusted.

The apparatus 1 for feeding light and heat may comprise a memory 48. In the memory 48, the temperature measured by the temperature sensor 50 when the subcutaneous temperature of the wearer of the apparatus 1 for feeding light and heat becomes appropriate is stored as the set temperature. The set temperature may be one value as an average value when the subcutaneous temperatures of many wearers are appropriate. It may be a different temperature for each wearer. Or, the set temperatures may be a plurality of different temperatures depending on the purpose of use of the apparatus 1 for feeding light and heat, such as lymphedema medical treatment, elimination of body stiffness due to lack of exercise, skin beauty, and the like. The controller 40 adjusts the amount of power supplied to the light source 16 for the human body and the light source 26 for temperature control so that the temperature measured by the temperature sensor 50 is the same as the set temperature. If the memory 48 is provided, the temperature switch 46 may not necessarily be provided.

By doing so, the subcutaneous temperature can be maintained at a suitable temperature, that is, a comfortable temperature by promoting blood circulation, based on the temperature measured by the temperature sensor 50. In particular, since the light source holder 20 for temperature control and the light source holder 10 for the human body are made of the same material, and the cavity 28 for temperature control and the cavity 18 for the human body are of the same shape and size, the energy received by the temperature sensor 50 from the light source 26 for temperature control is the same as the energy received by the skin from the light source 16 for the human body, and thus, the temperature measurement at the temperature sensor 50 is more consistent with wearer's subcutaneous temperature. Since the skin temperature and the subcutaneous temperature do not match, it is difficult to directly measure the subcutaneous temperature from the human body because of the large-scale equipment and the burden on the person whose temperature is to be measured. Furthermore, compared with the case of measuring the temperature of the skin, measuring the temperature with the temperature sensor 50 is has many advantages such as not being affected by perspiration and being able to reduce the equipment worn on the human body.

In the apparatus 1 for feeding light and heat shown in Fig. 1, the power source 30, the controller 40, the thermostat 44, the temperature switch 46, and the memory 48 are housed in a control box 42. Furthermore, the light source holder 20 for temperature control, i.e., the temperature sensor 50, is also housed in the control box 42. By this configuration, the apparatus 1 for feeding light and heat can be constructed by the light source holder 10 for the human body, or a sheet 60 with the light source holder 10 for the human body or a belt 70 with the light source holder 10 for the human body, which are below discussed, and the control box 42. So, handling it becomes easier. However, it is not limited to this configuration. It may also have either the temperature switch 46 or the memory 48. The controller 40, the temperature switch 46, the memory 48, etc., may be a part of another device such as a personal computer. Also, it doesn't have to be equipped with the thermostat 44.

Next, with reference to Fig. 4, an embodiment of fixing the light source holder 10 for the human body to the sheet 60 is discussed. The light source holder 10 for the human body may be fixed to the sheet 60 on either the human body side or the back side, but in Fig. 4 an example where it is fixed on the human body side is shown. As shown in Fig. 4(a), a plurality of the light source holders 10 for the human body are fixed to the sheet 60. Holes 62 are formed in the sheet 60 at locations corresponding to the cavities 18 for the human body of the plurality of the light source holders 10 for the human body so as not to block the light emitted from the light sources 16 for the human body. It is preferably hygienic to make the light source holder 10 for the human body, which contacts with the human body, replaceable. At that time, the sheet 60 may also be made replaceable. This is to keep the light source holder 10 for the human body clean after use or by exchanging the light source holder 10 for the human body for each wearer. Alternatively, a replaceable nonwoven fabric or paper 14 may be applied to the portion of the sheet 60 that contacts the skin. This is because the part that comes into contact with the human body is replaceable, and the nonwoven fabric or paper 14 is replaced after use or for each wearer, so that the sheet 60 can always be used in a clean state.

Fig. 4(b) shows an example for an arrangement of the light source holders 10 for the human body, that is, the light sources 16 for the human body. Arranging the light sources 16 for the human body in a grid pattern is suitable for evenly illuminating a wide area. The distance between the light sources 16 for the human body may vary depending on the purpose of the apparatus 1 for feeding light and heat. For medical treatment of lymphedema, for example, it may be 20 - 30 mm.

The sheet 60 is made of a material that has a little stretchability, but is flexible, such as polyvinyl chloride. The light source holders 10 for the human body are fixed to one side surface of the sheet 60 with a predetermined interval, and holes are formed at positions corresponding to the cavities 18 for the human body of the light source holders 10 for the human body. Besides, the cavities 18 for the human body may be arranged in multiple straight rows. In this case, the rear sides of the light source holders 10 for the human body arranged in a row (the side opposite to the side that contacts the human body) may be connected by means of a connector 66 that is harder to bend than the sheet 60. By using the connector 66, the sheet 60 with the light source holders 10 for the human body can be easily wrapped in a direction perpendicular to the longitudinal direction of the connector 66, for example, easily wrapped around an arm or a leg. Furthermore, by passing the cable for the LEDs 16 inside the connector 66, the cable is protected and does not get in the way during use. The material, shape, and the like of the connector 66 is not particularly limited as long as it has a rigidity that does not bend easily. For example, the connector 66 may be made of a vinyl chloride resin plate. It is preferable to attach a protective cover 68 that covers the plurality of the light sources 16 for the human body and the connector 66 so as to prevent damage to the cables. The sheet 60 with the light source holders 10 for the human body is wrapped around the human body and a string or band secures the sheet 60 from the outside.

Fig. 5 shows another example for an arrangement of the light sources 16 for the human body (the light source holders 10 for the human body) that is different from Fig. 4. The light sources 16 for the human body are placed in whorl from the center, the light source 16-1 for the human body. The term "whorl" refers to the state in which a single curve from the light source 16-1 for the human body goes around (counterclockwise in Fig. 5) and gradually moves away from the center. Also, when it goes around the center one time, the distance away from the center increases. By arranging the light sources 16 for the human body in this way, the light sources 16 for the human body may be arranged around the part where you want to irradiate the light strongly, such as the medical treatment part. Thus, the light can be emitted strongly at the center and gradually weaker around it. Furthermore, since the light sources 16 for the human body spread in whorl, a single cable (not shown) for supplying power to the light sources 16 for the human body can be easily placed in whorl to power multiple light sources 16 for the human body, and manufacturing becomes easier.

Note that the light sources 16 for the human body may be arranged concentrically. When they are arranged concentrically, the intervals between the concentric circles may be narrow near the center and widen away from the center, or may be equal. By arranging the light sources 16 for the human body concentrically, it is possible to irradiate the center with stronger light than the surroundings.

Next, with reference to FIG. 6, a belt 70 with the light source holder 10 for the human body, in which a plurality of the light source holders 10 for the human body are movably attached to the belt 70, is discussed. A slit 17 for passing the belt 70 is formed in the light source holder 10 for the human body. The slit 17 is formed closer to the back than the light source 16 for the human body so that it does not interfere with light irradiation from the light source 16 for the human body. The light source holder 10 for the human body is movably attached to the belt 70 by passing the belt 70 through the slit 17. The number of the light source holders 10 for the human body attached to the belt 70 is not limited, and any number of them may be attached according to the application.

Although the belt 70 may be made of any material, it is preferable to use a material that is strong enough to fix the light source holder 10 for the human body, is flexible, and has a specific gravity that is not too large. For example, a belt made of knitted cotton may be used. The belt 70 can be wrapped around, for example, an arm or leg to secure the light source holder 10 for the human body. At that time, since the light source holder 10 for the human body is movable on the belt 70, it can be moved so as to irradiate a desired position with strong light.

In the above discussion, it has been explained that a plurality of the light source holders 10 for the human body are fixed to the human body by being fixed to the sheet 60 or the belt 70. However, as shown in FIG. 7, for example, the light source holder 10 for the human body itself may widely spread, and a plurality of the cavities 18 for the human body are formed therein, and the light sources 16 for the human body are installed in the cavities 18, respectively. In this case, the main body of the light source holder 12 for a human body, which is the part of the light source holder 10 for the human body that connects the cavities 18 for the human body, may be made of, for example, chloroprene rubber so as to flex and deform along the complicated shape of a human body. In this case, the material around the cavity 18 for the human body and the material for the main body of the light source 12 for a human body may be the same or different, that is, different materials may be combined to form the light source holder 10 for the human body.

Alternatively, the light sources 16 for the human body arranged in a row as shown in Fig. 3 may be installed in one the light source holder 10 for the human body. In this case, the light source holder 10 for the human body does not need to bend, and the connector 66 does not need to be provided if the light source holder 10 for the human body does not bend.

Next, the effect of irradiating the human body with light of near-infrared rays is discussed. When the light of near-infrared rays is applied to the human body from the light source 16 for the human body, the light energy reaches the depth of 2 to 6 mm under the skin. Since the superficial lymph capillaries are around 50 µm deep, the deep lymphatics are around 100 - 200 µm, and there are slightly thicker lymphatic vessels and blood vessels behind them, all of them are within the range of near-infrared rays irradiation. Cytochrome C oxidase is activated by the light of near-infrared rays. Activation of cytochrome C oxidase increases energy produced by cells and promotes metabolism. Furthermore, if the skin is warmed to a temperature of, for example, 40-42°C, which improves subcutaneous blood circulation, the supply of nutrients and oxygen on the arterial side and the reabsorption of bodily fluids on the venous side will increase. Hypothermia and hypoxia at the tissue level caused by long-term internal pressure stress, which is considered to be one of the aggravating factors of edema, are improved, and thus, fundamental medical treatment of lymphedema can effectively be performed.

In the experience of the inventors, among near-infrared rays, light with a wavelength of 800 to 820 nm is effective in activating cytochrome C oxidase. Thus, it is preferable to irradiate light having a wavelength distribution with a peak of 800 nm to 820 nm.

As discussed above, by the apparatus 1 for feeding light and heat of the present invention, light of near-infrared rays can be given to the subcutaneous region and the temperature can be increased to improve blood circulation, so that lymphedema can be medically treated. Furthermore, activation of subcutaneous cytochrome C oxidase and promotion of blood circulation provide many effects, such as recovery of physiological functions and skin rejuvenation.

### Working Example

An example in which the apparatus for feeding light and heat of the invention is applied to a patient with lymphedema is discussed below. The conditions to use the apparatus were as follows.

### Apparatus for feeding light and heat

A sheet with light source holders for a human body, in which 6×6, i.e., 36 LEDs with a rated wavelength of 800 nm are arranged on a lattice sheet at intervals of 3 cm was used. For an ankle, a belt with light source holders for a human body, in which 12 LEDs with a rated wavelength of 800 nm are arranged at equal intervals of 3 cm, was used. The temperature measured by the temperature sensor was controlled to be 40 °C.

### Conditions for each use

A group of proximal lymph nodes (groin), proximal strong fibrosis site (focusing on the site of concentrated tissue fibrosis that occurs in the thigh), a group of distal lymph nodes (popliteal, ankle), and distal strong fibrosis site (calf, etc.) were irradiated with light for 20 to 25 minutes.

### Frequency of use

The light irradiation as above-mentioned was performed twice a week from July 27, 2020 to February 9, 2021.

### Effects of use

A patient with lymphedema had symptoms such as swelling of the entire lower extremity, especially of the left leg, progressing fibrosis of tissue, and inability to bend the knees and ankles, due to excessive body fluid. By the above-mentioned use for about six and a half months, the swelling was improved, tissue fibrosis was alleviated, and the knees and ankles became able to bend. Fig. 8 shows the measuring results of the thickness (cm) of the left foot at the groin on the vertical axis and the dates of the measurements on the horizontal axis. It can be understood from the figure that the swelling was remarkably reduced.

The major symbols used in the present specification and drawings are listed below.
- 1: apparatus for feeding light and heat
- 10: light source holder for a human body
- 12: main body of light source holder for a human body
- 14: nonwoven fabric
- 16: a light source for the human body (LED)
- 16-1: LED placed at the center of whorl
- 17: slit
- 18: cavity for the human body
- 19: LED shelf
- 20: light source holder for temperature control
- 26: light source for temperature control
- 28: cavity for temperature control
- 30: power source
- 32: battery
- 40: controller
- 42: control box
- 44: thermostat
- 46: temperature switch
- 48: memory
- 50: temperature sensor
- 60: sheet
- 62: hole
- 66: connector
- 68: protective cover
- 70: belt

## Claims

1. An apparatus for feeding light and heat comprising:
a light source holder for a human body that can be fixed in contact with the human body, and has a cavity for the human body that is recessed from a side that contacts the human body;
a light source for the human body that is installed in the cavity for the human body and emits light of near-infrared rays;
a light source holder for temperature control, wherein a cavity for temperature control is formed from one side, wherein the light source holder for temperature control is formed of the same material as the light source holder for the human body, and wherein the cavity for temperature control has the same shape as the cavity for the human body;
a light source for temperature control installed in the cavity for temperature control and emitting light of the same wavelength as the light source for the human body;
a temperature sensor installed at the entrance of the cavity for temperature control of the light source holder for temperature control and covering the entrance;
a power source that supplies a same power to the light source for the human body and the light source for temperature control;
a temperature switch that is operated when subcutaneous temperature reaches a suitable temperature; and
a controller that adjusts power supplied from the power source to the light source for the human body and the light source for temperature control based on a temperature measured by the temperature sensor,
wherein the controller controls the power supplied to the light source for the human body and the light source for temperature control so that the temperature measured by the temperature sensor is maintained at the temperature when the temperature switch is operated.

2. An apparatus for feeding light and heat comprising:
a light source holder for a human body capable of being fixed in contact with a human body, wherein a cavity for the human body that is recessed from a side in contact with the human body is formed;
a light source for the human body that is installed in the cavity for the human body and emits light of near-infrared rays
a light source holder for temperature control, wherein a cavity for temperature control is formed from one side, wherein the light source holder for temperature control is formed of the same material as the light source holder for the human body, and wherein the cavity for temperature control has the same shape as the cavity for the human body;
a light source for temperature control installed in the cavity for temperature control and emitting light of the same wavelength as the light source for the human body;
a temperature sensor that is located at an entrance of the cavity for temperature control of the light source holder for temperature control and that covers the entrance;
a power source providing a same power to the light source for the human body and the light source for temperature control;
a memory for storing a set temperature, which is a target temperature of a temperature measured by the temperature sensor; and
a controller that adjusts power supplied from the power source to the light source for the human body and the light source for temperature control based on the temperature measured by the temperature sensor,
wherein the controller adjusts the power supplied to the light source for the human body and the light source for temperature control to maintain the temperature measured by the temperature sensor at the set temperature.

3. An apparatus for feeding light and heat of Claim 1 or 2,
wherein the light of the same wavelength has a peak wavelength of 800 nm to 820 nm.

4. An apparatus for feeding light and heat of Claim 1 or 2,
wherein the light source holder for a human body is made of silicon rubber and is replaceable.

5. An apparatus for feeding light and heat of Claim 1 or 2,
wherein the apparatus has a plurality of the light source holders for the human body, the plurality of light source holders for the human body being fixed to a sheet and arranged two-dimensionally.

6. An apparatus for feeding light and heat of Claim 5,
wherein The plurality of light source holders for the human body are arranged in whorl.

7. An apparatus for feeding light and heat of Claim 5,
wherein the plurality of light source holders for the human body are arranged in a lattice pattern, and
wherein the light source holders for the human body in a same row are connected by a connector that is less bendable than the sheet.

8. An apparatus for feeding light and heat of Claim 1 or 2,
wherein a plurality of cavities for the human body are formed in the light source holder for the human body, and
wherein each cavity for the human body is provided with the light source holder for the human body.

9. An apparatus for feeding light and heat of Claim 1 or 2,
wherein the apparatus has a plurality of the light source holders for the human body, the plurality of light source holders for the human body being movably attached to a belt.
